# EUROPÄISCHE PATENTSCHRIFT

(11) **EP 3 393 413 B1**
(45) Veröffentlichungstag und Bekanntmachung des Hinweises auf die Patenterteilung: **26.05.2021**
(21) Anmeldenummer: 16819554.3
(22) Anmeldetag: 21.12.2016
(51) Int. Cl.: A61F 13/00, A61F 13/02, A61F 13/08

(54) **KOMPRESSIONSBINDENZUSAMMENSTELLUNG**
COMPRESSION BANDAGE COMBINATION
COMBINAISON DE BANDAGES COMPRESSIFS

(30) Priorität: 21.12.2015 DE 102015226368
(43) Veröffentlichungstag der Anmeldung: 31.10.2018
(73) Patentinhaber: KOB GmbH, 67752 Wolfstein (DE)
(72) Erfinder: LANGEN, Guenter, 67752 Wolfstein (DE)
(74) Vertreter: DREISS Patentanwälte PartG mbB
(86) Internationale Anmeldenummer: PCT/EP2016/082219
(87) Internationale Veröffentlichungsnummer: WO 2017/108980

(56) Entgegenhaltungen:
- EP-A1- 2 698 135
- EP-A2- 0 878 179
- EP-A2- 2 275 062
- DE-A1-102010 042 772
- US-A1- 2012 238 933

## Beschreibung

Kompressionsbindenzusammenstellung umfassend eine erste innere Binde sowie eine zweite äußere Binde, wobei die zweite äußere Binde im Gebrauch über die erste innere Binde anlegbar ist.

Derartige mehrlagige Verbände (Multilayerverband) zur Verwendung als Kompressionsverband am menschlichen oder tierischen Körper sind bekannt. Die Verbände werden dabei durch Übereinanderwickeln von mindestens zwei separaten unterschiedlichen Binden am Körperteil angefertigt. Dabei ist es bekannt, die innere Binde als Polsterbinde zu gestalten, die direkt auf die Haut des Körperteils als innere Lage angewickelt wird und darüber eine Kompressionsbinde zu platzieren, die im Anschluss an die erste innere Binde als äußere Lage über diese gewickelt wird, wobei beide Lagen aufeinander haften und eine rutschsichere Verbindung eingehen.

So ist es beispielsweise aus dem Stand der Technik bekannt, z. B. aus der EP 1 709 947, ein Kompressionsbandagensystem einzusetzen mit einer inneren hautzugewandten elastischen Bandage, die ein elastisches Substrat sowie eine gedehnte Schaumschicht umfasst, die mindestens 33% in Querrichtung und 67% in Längsrichtung des elastischen Substrates überdeckt sowie einer zweiten gedehnten selbsthaftenden elastischen Bandage, die Kompressionskräfte aufweist, wenn sie gedehnt wird. Eine ähnliche Ausgestaltung ist aus der EP 2 275 062 A2 bekannt.

Weiterhin ist eine Bandage zum Anlegen an einen menschlichen oder tierischen Körper aus der DE 10 2010 042 772 A1 bekannt, die aus einem textilen Material besteht, wobei aus der Grundstruktur eine Polsterschicht aus Fäden oder Fasern herausgezogen ist bzw. in diese eingebracht ist.

Ein weiteres zweilagiges Kompressionsbandagensystem ist aus der US 2012/0238933 A1 vorbekannt.

Ausgehend von diesem Stand der Technik hat sich die Erfindung die Aufgabe gestellt, die genannte Zusammenstellung aus Kompressionsbinden sowohl hinsichtlich der Polsterwirkung als auch hinsichtlich der Anlegesicherheit zu verbessern.

Die Erfindung betrifft eine Kompressionsbindenzusammenstellung mit den Merkmalen des Anspruchs 1, bei der die erste innere Binde einen ersten Abschnitt umfasst, der zumindest auf seiner der Haut eines Trägers zugewandten Seite eine Polsterschicht aufweist und einen zweiten an den ersten Abschnitt anschließenden elastischen und kohäsiv haftenden Abschnitt, wobei die zweite äußere Binde elastisch und kohäsiv haftend ausgebildet ist. Dabei wird durch die Erfindung eine rutschsichere Verbindung aufgrund des kohäsiv haftenden zweiten Abschnitts der inneren Binde und der hiermit zusammenwirkenden ebenfalls kohäsiv haftend ausgebildeten äußeren Binde bereitgestellt.

Dabei umfasst die äußere Binde mindestens zwei miteinander verbundene Bindenlagen (Flächengebilde), die im separierten Zustand voneinander verschiedene elastische Eigenschaften aufweisen. Dabei kann durch die Kombination zweier Bindenlagen mit verschiedenen Dehnungseigenschaften erreicht werden, dass zum einen eine Dehnungsbegrenzung eingestellt und bei günstiger Kombination die Kompression auch bei niedriger Dehnung vorteilhaft eingestellt werden kann. Besonders bevorzugt vermeidet dabei eine Dehnungsbegrenzung Wickelfehler, die bei Anwickeln einer Binde mit hoher Dehnfähigkeit entstehen können und dann zu gesundheitlichen Problemen wie Abschnürungen, Durchblutungsstörungen oder Nekrosen führen können.

Dabei kann vorgesehen sein, dass auf der der Haut zugewandten Seite der Polsterschicht der ersten inneren Binde eine diskontinuierliche, insbesondere offenporige Kunststoffbeschichtung aufgebracht ist. Auf diese Weise wird erreicht, dass eine Rutschhemmung auf der Hautseite erzielt werden kann, die die innere Binde in der gewünschten Position auf der Haut eines Trägers fixiert. Durch die offenporige Beschichtung wird weiterhin eine Atmungsaktivität erreicht. Es kann jedoch auch vorgesehen sein, die Beschichtung als geschlossenen Film auszuführen. Die offenporige Beschichtung kann insbesondere nach folgenden Verfahren erfolgen:
a) Es kann ein Sprühauftrag von Polymerdispersionen, wie Naturlatex, Synthesekautschuklatices sowie aus den Rohstoffen Butadien, Styrol, Acrylnitril, Acrylsäureester, Polyurethan, Polysiloxane und deren Copolymere sowie Kombinationen hiervon.
b) Ein Sprühauftrag von Schmelzklebstoffen auf Basis von Synthesekautschuken, Polyurethanen, Polyacrylaten und/oder Silikonkautschuken.
c) Aufstreuen eines Kunststoffpulvers und anschließend Anschmelzen des Kunststoffpulvers bei einer Partikelgröße von 100 bis 500 microns auf Basis thermoplastischer Kunststoffe oder Elastomere, z. B. PVAc, PAC, PE, PP, PES, IR.
d) Aufbringen mittels Schablonendruck unter Verwendung verdickter Polymerdispersionen, wie unter a) genannt oder mit Schmelzklebstoffen wie unter b) genannt.
e) Rasterwalzenauftrag unter Verwendung verdickter Polymerdispersionen, wie unter a) genannt oder mit Schmelzklebstoffen, wie unter b) genannt.
f) Offenporiger Auftrag von verschäumten Polymerdispersionen, wie unter a) genannt.

Die genannten Verfahren werden so ausgeführt, dass nach der Beschichtung, Trocknung und dem Abkühlen ein mikroporöser, offenporiger Auftrag von Kunststofffragmenten in einer Auftragsmenge von vorzugsweise 2 bis 20 g/m² (trocken) entsteht, je nach Oberflächenstruktur. Die Kunststofffragmente sind dabei bevorzugt unmittelbar an der Oberfläche angeordnet und haftend mit den Fasern der Polsterschicht verbunden. Überraschenderweise wurde dabei gefunden, dass bereits eine geringe Menge der Kunststofffragmente in dem angegebenen Auftragsmengenbereich genügt, um einen rutschfesten Sitz am Körperteil zu erreichen. Hierdurch wird auf der einen Seite eine kostengünstige Variante bereitgestellt, die gleichzeitig positiv für die Atmungsaktivität sowie den Tragekomfort des Verbandes ist. Dies gilt insbesondere, da derartige mehrlagige Kompressionsverbände üblicherweise als Dauerverbände über mehrere Tage bis zwei Wochen getragen werden. Hierbei ist während der gesamten Tragedauer der möglichst optimale Sitz des Verbandes wichtig für den Heilungserfolg der Kompressionsbehandlung.

Dabei kann besonders bevorzugt sein, dass die beiden Abschnitte der inneren Binde miteinander verbunden sind und sich in Längsrichtung der ersten Binde aneinander anschließen. Neben dieser bevorzugten Ausführungsform kann jedoch auch vorgesehen sein, dass die beiden Abschnitte der inneren Binde unverbunden bereitgestellt werden. In aller Regel werden die beiden Abschnitte aufgewickelt dargeboten, indem diese auf einen Hülsenkern aus Hartpappe oder Kunststoff aufgewickelt sind, wobei der erste Abschnitt im aufgewickelten Zustand außen zu liegen kommen sollte, da er zuerst auf die Haut eines Patienten aufgewickelt wird.

Besonders bevorzugt weist der erste Abschnitt eine rechteckige Gestalt mit einer Breite von 60 bis 150 mm und einer Länge von 150 bis 300 cm auf. Hieran schließt sich der zweite Bereich an, der ebenfalls die Gestalt eines rechteckigen Flachmaterials mit einer Breite von 60 bis 150 mm und einer Länge von 150 bis 300 cm aufweisen kann, wobei die Länge im gedehnten Zustand angegeben ist. Die Verbindung der beiden Abschnitte kann über Nähen, Kleben, Laminieren, Heißschweißen, Ultraschallschweißen, etc. erfolgen, wobei sowohl eine Verbindung der beiden Abschnitte auf Stoß oder überlappend im Bereich einer Schmalseite erfolgen kann, so dass die beiden Abschnitte zu einem einzigen Streifen verbunden sind. Dabei wird an die Festigkeit der Verbindung nur die Anforderung gestellt, dass die Zugbelastung beim Anwickeln der Binde eine zerstörungsfreie Applikation am Körperteil erlaubt. Darüber hinaus ist es vorteilhaft, wenn die Verbindungsstelle im Wesentlichen nicht steifer bzw. dicker als die übrige Binde ist. Die Abschnitte werden derart zu einem zylindrischen Bindenkörper aufgewickelt, dass der erste Abschnitt die äußere Wicklung bildet und im Falle einer dort aufgebrachten Kunststoffbeschichtung die Antirutscheigenschaften bereitstellt und diese vorzugsweise die äußere Lage bildet, so dass beim Anwickeln die Kunststoffbeschichtung auf der Haut eines Trägers zu liegen kommt und ein einfaches Wickeln sichergestellt ist.

Dabei kann vorgesehen sein, dass die Polsterschicht als Polsterwatte, insbesondere aus den Fasermaterialien Baumwolle, Viskose, Polyester, Polypropylen oder Mischungen aus diesen Faserstoffen ausgebildet ist. Das Flächengewicht der Polsterwatte liegt vorzugsweise im Bereich von 50 bis 150 g/m², bevorzugt von 60 bis 120 g/m² und besonders bevorzugt von 80 bis 100 g/m², wobei die Polsterwatte zur Bildung der Schicht verdichtet sein kann, z. B. über Kalandrieren oder Vernadeln. Bevorzugt zeigt der erste Abschnitt mit der Polsterschicht keine Elastizität bzw. Rückzugsvermögen in Längsrichtung, sondern allenfalls eine geringe Dehnfähigkeit von 10 bis 20% gemessen nach DIN 61632, die für ein faltenfreies Anlegen am Körperteil ausreichend ist.

Auf einer Oberfläche der Polsterwatte kann dann die Kunststoffbeschichtung in Form von Kunststofffragmenten zur Gewährleistung eines rutschfesten Sitzes bei hautseitiger Wicklung am Körperteil bereitgestellt werden. Die Polsterschicht schützt dabei der Körperteil, an dem die Binden angelegt werden, gleicht Unebenheiten, insbesondere an Knochenerhebungen aus und schützt die jeweilige Extremität bei Anlegen einer solchen.

Der zweite Abschnitt der ersten inneren Binde umfasst eine in Längsrichtung elastisches, kohäsiv selbsthaftendes Flächengebilde, insbesondere auf Basis eines Gewebes, Gewirkes oder Vlieses, wobei die Elastizität durch die Einarbeitung von elastischen Fäden, wie beispielsweise hochgedrehte Baumwollfäden, als Spin- oder Zwirnkreppfäden, texturierte Polyamid- oder Polyestergarne, Gummi- bzw. Polyurethan-Elastanfäden hervorgerufen werden kann.

Dabei kann vorgesehen sein, die erste innere Binde in Spiraltouren an einem Körperteil anzuwickeln, wobei das Anwickeln so erfolgt, dass bei vorgesehener Antirutschbeschichtung diese unmittelbar auf dem Körperteil zu liegen kommt. Wird die Verbindungsstelle oder der Überzug des ersten und des zweiten Abschnitts erreicht, wird erfindungsgemäß einfach weitergewickelt bzw. der zweite Bindenabschnitt angesetzt, wobei nun die Wicklung weiterhin in Spiraltouren unter Zug und Ausnutzung der Längselastizität des zweiten Abschnitts fortgesetzt wird, bis die Polsterschicht des ersten Abschnitts vollständig durch den zweiten Abschnitt abgedeckt ist. Dadurch wird die Polsterschicht an der Extremität sicher gehalten und der zweite Abschnitt übt nach Applikation eine leichte bis mittlere Kompression aus, wobei ein bevorzugter Kompressionsdruck von ca. 10 bis 20 mm/Hg am B-Maß gemäß P.D. Asmussen, B. Söllner: Kompressionstherapie, Urban und Fischer, 2004, Seite 133-134 gemessen wird. Bei der Messstelle B handelt es sich um eine Stelle, die den Fesselbereich eines menschlichen Beins betrifft Die Messstelle wird gemäß der heranzuziehenden Vorschrift RAL-GZ 387 bestimmt. Der durch die erfindungsgemäße Binde auf das Bein ausgeübte Kompressionsdruck wird unter Verwendung des Meßgeräts, Typ PICOPRESS , ermittelt, indem der Drucksensor des Picopress-Geräts unmittelbar über dem Knöchel am sog. B-Maß nach RAL - GZ 387 aufgeklebt bzw. befestigt wird und darüber dann die Binde wie beschrieben angewickelt wird. Nach Aktivierung des Sensors kann dann der von der Binde ausgeübte Kompressionsdruck auf die B-Maß- Stelle in mm Hg abgelesen werden. Dabei entspricht die Messstelle B einem Punkt am menschlichen Unterschenkel, der unmittelbar über dem Knöchel an dem Punkt mit dem geringsten Umfang des Unterschenkels liegt.

Aufgrund der Kohäsivität des zweiten Abschnitts haften die Lagen aufeinander und stellen einen rutschfesten Sitz der ersten inneren Binde am Körperteil sicher. Zudem ermöglicht die kohäsiv haftende Außenlage der inneren Binde die Verankerung mit der im nächsten Schritt erfindungsgemäß anzulegenden zweiten äußeren Binde.

Die zweite äußere Binde besteht aus einer elastischen, kohäsiven selbsthaftenden Binde vorzugsweise auf Basis eines textilen Verbundmaterials, wobei dieses zwei elastische, kohäsive selbsthaftende textile Bindenlagen umfasst, die vorzugsweise als textile Flächengebilde ausgebildet sind, wobei die beiden Binden vorzugsweise einander vollflächig überdecken und insbesondere kantengenau verlaufen, so dass diese entlang ihrer gesamten Länge und Breite miteinander haftend verbunden sind über die bestehenden kohäsiven Kräfte.

Die Haftkräfte des kohäsiv haftenden Bindenabschnitts sowie der zweiten Binde sind so gestaltet, dass ein Verrutschen der Bindenlagen aufeinander vermieden ist. Kohäsive Haftung bedeutet, dass keine Anhaftung an z. B. Haut oder Bekleidung erfolgt, sondern die Haftung nur zwischen den Bindenlagen (Oberfläche zu Oberfläche) besteht.

Die Bestimmung der Haftkräfte erfolgt dabei nach dem nachfolgend beschriebenen Verfahren:
Die Haftkraft ist die ermittelte Kraft, die für das Trennen von kohäsiven Proben im sog. 180 ° T-Peel- Test benötigt wird.

Das kohäsive beschichtete Textil wird spannungslos und faltenfrei ausgelegt. Daraus wird eine 10cm breite und 40cm lange Probe geschnitten. Der 40cm lange Probestreifen wird in der Mitte in 2 Streifen der Länge 20 cm durchgeschnitten Die beiden 20cm langen Streifen werden so aufeinander gelegt, dass die Seite A des ersten Streifens auf der Seite B des zweiten Streifens liegt. Die so vorbereitete Probe wird auf eine beheizte Edelstahlplatte (40 °C) gelegt und mit einer beheizten Metallwalze (40 °C) insgesamt 40-mal innerhalb von 30 sec gewalzt (20x hin und zurück).

Das Gewicht der Metallwalze beträgt 8 kg für 10 cm Probenbreite , d.h. 0,8 kg je cm Probenbreite für andere Breiten als 10 cm.

Anschließend wird die Kraft zur Trennung der Lagen der Probe in einem Kraft-Dehnungs-Prüfgerät (Hersteller: ZWICK , INSTRON) ermittelt . Dazu wird das Ende der ersten Lage in der unteren Klemme und das andere Ende der zweiten Lage in der oberen Klemme eingespannt, wobei darauf geachtet wird, dass die Probe möglichst ohne Verzug, d.h. spannungsarm, zwischen den Klemmen positioniert ist, damit keine "Ruhekraft" anliegt. Diese Anordnung entspricht einem 180° T-Peel- Test. Zur Messung bewegen sich die Klemmen vertikal auseinander, wobei die dabei wirkende Trennkraft (entspricht der Momentan- Haftkraft der Probe) kontinuierlich registriert wird. Durch Integration der Kraft über den zurückgelegten Weg der Klemmen wird die Trennarbeit und daraus die mittlere Trennkraft = Haftkraft in cN/cm erfasst und berechnet. Diese Haftkraft entspricht den Zahlenangaben bei den Beispielen.

Dabei ist es besonders vorteilhaft, wenn die Haftkraft des kohäsiv haftenden Bindenabschnitts sowie der kohäsiv haftenden zweiten Binde 20-150 cN/cm, weiter bevorzugt 30-100 cN/cm und weiter bevorzugt 40-80 cN/cm beträgt.

Die einzelnen Bindenlagen sind dabei vorzugsweise so ausgestaltet, dass sie verschiedene Dehnbarkeiten aufweisen, und zwar vorzugsweise eine Dehnbarkeit von 100 bis 180% für eine Lage und für die andere Lage eine Dehnbarkeit von 10 bis 100%. Je nach Dehnbarkeit werden die Binden den Kategorien Kurz-, Mittel- oder Langzugbinde zugeordnet, wie in P. Asmussen, B. Söllner, Kompressionstherapie Prinzipien und Praxis, Verlag Urban & Fischer in Elsevier, 2004 auf Seite 121 beschrieben. Die Dehnbarkeiten werden dabei nach DIN 61632 bestimmt. Dabei handelt es sich bei der einen Bindenlage um eine Bindenlage vom Typ einer Langzugkompressionsbinde, wobei hierzu vorzugsweise kohäsiv haftende elastische Vliesbinden, wie sie handelsüblich unter dem Namen Coban®, Hersteller 3M bzw. Lastopress®, Hersteller Paul Hartmann AG, Heidenheim, Deutschland erhältlich sind, verwendet werden können. Bei diesen Binden wird die Dehnbarkeit in Längsrichtung durch Einarbeiten (Vernähen, Verkleben, Ultraschallschweißen) von elastischen Fäden aus Gummi oder Elastan in ein starres Tägervlies erreicht.

Bei der zweiten Bindenlage handelt es sich um eine Bindenlage vom Typ Kurzzugbinde oder Mittelzugbinde auf Basis vorzugsweise eines Trägertextils aus Gewebe, Gewirke oder Nonwoven, welches starr oder in Längsrichtung dehnbar sein kann. Im letzteren Fall wird die Dehnbarkeit in Längsrichtung insbesondere durch das Einarbeiten von elastischen Fäden (hochgedrehten Baumwollfäden als Spin- oder Zwirnkreppfäden, texturierte Polyamid- oder Polyestergarne, Gummi- bzw. Polyurethan-Elastanfäden oder ähnliches) hervorgerufen. Je geringer die Dehnbarkeit, desto mehr nähert man sich an eine starre Bindenkonstruktion (ohne Dehnbarkeit bzw. Elastizität) an. Darüber hinaus kann vorgesehen sein, dass die beiden Bindenlagen so miteinander verbunden sind, dass sich eine Dehnbarkeit der zweiten äußeren Binde gemessen nach DIN 61632 bei Standardlaborbedingungen von D_{fix} von 40 bis 100% ergibt. Beim Anlegen der Binde von Hand kann die zweite äußere Binde maximal auf den Wert D_{fix} gedehnt werden, d. h. die Dehnung kann keine höheren Werte als D_{fix} annehmen, so dass eine Dehnungsbegrenzung bereitgestellt wird. Durch die Dehnungsbegrenzung wird erreicht, dass die zweite äußere Binde dem Typ einer Kurzzugbinde oder Mittelzugbinde entspricht, obwohl im Verbundmaterial aus den mindestens zwei Bindenlagen eine Binde vom Typ einer Langzugbinde integriert sein kann. Hierdurch wird der Vorteil erreicht, dass die Kompressionsdruckwirkung der zweiten äußeren Binde bei maximaler Dehnung D_{fix} signifikant niedriger ist als sie es für die erste Bindenlage alleine wäre.

Besonders vorteilhaft dabei ist, dass im Herstellungsverfahren der zweiten äußeren Binde die Dehnung D_{fix} innerhalb eines Bereichs eingestellt werden kann, vorzugsweise in einem Bereich von 40 bis 100%, wobei die Einstellung variabel auf einen beliebigen gewünschten Zielwert erfolgen kann. Durch die Dehnungsbegrenzung ergibt sich dann auch eine korrespondierende Begrenzung der Kompressionsdruckwirkung. Idealerweise liegt der Kompressionsdruck der zweiten äußeren Binde gemessen am B-Maß zwischen 15 und 40 mm Hg.

Die Herstellung der erfindungsgemäßen äußeren zweiten Binde erfolgt bevorzugt unter Verwendung getrennter Bahnen der mindestens beiden Bindenlagen. Dabei wird die erste Bindenlage mit der größeren Dehnbarkeit vorzugsweise mit Hilfe eines separat angetriebenen ersten Quetschwalzenpaares mit einer ersten Transportgeschwindigkeit abgezogen, wobei möglichst geringe Zugkräfte verwendet werden, um den ungedehnten Zustand der Bindenlage weitgehend zu erhalten. Vorzugsweise ist die Breite der Quetschwalzen mindestens so breit wie die Bahnbreite der jeweiligen Bindenlage.

Vorzugsweise mit Hilfe eines angetriebenen zweiten Quetschwalzenpaares wird die zweite Bindenlage der zweiten kohäsiv haftenden Bindenlage getrennt von der ersten kohäsiv haftenden Bindenlage mit einer zweiten Transportgeschwindigkeit eingezogen, wobei die Bahnen im Wesentlichen kantengerade übereinander in der gleichen Transportrichtung geführt werden. Weitere Bahnen werden entsprechend zugeführt.

Dabei dienen die Quetschwalzenpaare als Klemmpunkte, so dass der Transport der Materialbahnen schlupffrei erfolgen kann.

Die Bahnen der Bindenlagen werden anschließend zwischen einem angetriebenen dritten Quetschwalzenpaar kantengerade zusammengeführt und miteinander zu einem Lagenverbund haftend verpresst, wobei insbesondere die Geschwindigkeit der zweiten Bindenlage größer ist als der ersten Bindenlage. Dadurch wird ein Verzug der Bahnen in Längsrichtung auf den gewünschten Dehnungszustand D_{fix} vorzugsweise im Bereich von 40 bis 80% erreicht, so dass die erfindungsgemäße Dehnungsbegrenzung der zweiten Binde sichergestellt werden kann.

Fakultativ kann ein weiteres angetriebenes Quetschwalzenpaar eingebaut sein, um die Klemmung der Bindenlage der zweiten äußeren Binde zu verstärken und einen schlupffreien Transport zu erreichen, falls dies durch das bereits vorgesehene Quetschwalzenpaar nicht hinreichend erreicht werden kann. Nach dem Verbinden der Lagen wird der Verbund aus den Bindenlagen durch ein weiteres angetriebenes Quetschwalzenpaar geführt, welches so eingerichtet wird, dass die Transportgeschwindigkeit im Wesentlichen wieder der Ursprungsgeschwindigkeit entspricht. Dadurch wird der Verzug der verbundenen Bahnen (zweite äußere Binde) aufgehoben und der ursprünglich ungedehnte Zustand wiederhergestellt. Je nach Art der zweiten Bindenlage und deren Dehnbarkeit sowie dem eingestellten Dehnungszustand D_{fix} weist die zweite äußere Binde nach dem ersten Quetschwalzenpaar, durch das die Bindenlagen hindurchgeführt werden, eine mehr oder weniger glatte oder gewellte Struktur auf. Je starrer die zweite Bindenlage ist, desto höher ist die Wellung des Verbundes, da die Stauchung stärker ausfällt

Durch die Zusammenstellung der mindestens zwei Binden wird das technische Problem einer kostengünstigen Kompressionsbindenzusammenstellung aus zwei getrennten Binden, wobei die Binden im angelegten Zustand aufeinander haften und eine rutschsichere Verbindung eingehen, gelöst. Darüber hinaus löst die Kompressionsbindenzusammenstellung das Problem des Herabrutschens am Bein während des Tragens als Dauerverband.

Gelöst wird dies durch die Kombination zweier unterschiedlicher textiler Flächengebilde, nämlich der ersten und der zweiten Binde. Durch Einstellung eines maximalen Dehnungszustands Dₘₐₓ wird darüber hinaus die Gefahr von Fehlanlegungen verringert.

Dabei können die Binden der Kompressionsbindenzusammenstellung einzeln konfektioniert und angeboten werden oder auch einem Set zusammen verpackt sein und dargeboten werden.

Im Folgenden soll nun ein bevorzugtes Ausführungsbeispiel näher erläutert werden.

### Beispiel:

### Erste innere Binde:

Die innere Binde wird aus einem ersten Abschnitt L1 und einem zweiten Abschnitt L2 zusammengesetzt. L1 umfasst eine Polsterwatte aus dem Fasermaterial Polyester, wobei die Fasern durch Vernadelung zu einem stabilen Vliesstoff verfestigt sind. Das Flächengewicht der Polsterwattebahn beträgt 80 g/m², die Dicke der Bahn beträgt 3 mm. Die Polsterwatte bildet damit eine Polsterschicht in Gestalt eines rechteckigen Streifens mit einer Breite von 100 mm und einer Länge von 250 cm (ungedehnt). Der Abschnitt L1 wird auf einem festen Untergrund vorgelegt und ist dann für die einseitige rutschhemmende Beschichtung vorbereitet. Für die rutschhemmende Beschichtung wird eine wässrige Polymerdispersion vom Typ Butadien-Styrol-Copolymerisat , Handelsname ACRONAL S 560, Hersteller BASF, Ludwigshafen, Deutschland, verwendet. Diese Dispersion hat einen Feststoffgehalt von 50%, die Viskosität beträgt 100 mPas, der pH-Wert 8,0. Die Dispersion wird unverdünnt bei Raumtemperatur von 20 bis 25° C in eine Drucklufthandsprühpistole gefüllt. Die Düse wird nach Öffnen des Ventils in einem Abstand von 30 bis 50 cm entlang der Oberfläche des ersten Abschnitts der ersten inneren Binde geführt und dabei die Dispersion durch den Druckluftstrahl in Gestalt eines feinen Tropfensprühnebels in möglichst gleichmäßiger Flächenverteilung auf die Oberfläche des Abschnitts aufgetragen. Durch Wägung des Abschnitts vor und nach dem Sprühauftrag wird ermittelt, dass der Nassauftrag bei 32 g/m² liegt. Der nasse Streifen wird anschließend mit einem Heißluftgebläse oder einer Infrarotlampe getrocknet. Durch Wägung wird ermittelt, dass der Beschichtungsauftrag trocken bei 11 g/m² liegt. Das getrocknete Butadien-Styrol-Copolymerisat haftet in Gestalt mikropunktueller, feiner Fragmente (Tropfen) an den Fasern der Oberfläche des Abschnitts und bewirkt die rutschhemmende Wirkung. Im Anschluss an den Abschnitt L1 wird in Längsrichtung der Abschnitt L2 gleichfalls in Gestalt eines rechteckigen Streifens der Breite 100 mm angelegt. Die Länge des Abschnitts L2 beträgt 200 cm gedehnt.

Der Abschnitt L2 umfasst eine in Längsrichtung elastische, kohäsive selbsthaftende Binde auf Basis eines offenporigen Fixierbindengewebes vom Typ 180, Handelsname RAPIDELAST cohesive, Hersteller KARL OTTO BRAUN GmbH, Wolfstein, Deutschland, der nachstehend spezifiziert ist.

30 % Baumwolle, 45 % Polyamid , 25% Kautschuk (NR) Kettfaden - Material 78 dtex f 17 x 2 Polyamid texturiert Fadenzahl / Kettdichte 110 pro 10 cm Breite Schussfaden - Material 17 tex Baumwolle braun Fadenzahl / Schussdichte 68 pro 10 cm gedehnt (DIN 61632) Flächengewicht gedehnt 41 g/qm Elastizität in Längsrichtung (Kettrichtung) Dehnbarkeit / Rückzug nach DIN 61632 100 % / 99 % Luftdurchlässigkeit DIN 53887 6000 l/qm sec Haftkraft Seite A/B 50 cN/cm

Zum Verbinden der Abschnitte L1 und L2 wird auf die nichtbeschichtete Oberfläche des Abschnitts L1 im Abstand von 5 mm vom Streifenende eine 1 mm breite Klebstoffraupe über die gesamte Bindenbreite von 100 mm aus einer handelsüblichen Heißklebepistole aufgetragen. Das Ende des Abschnitts L2 der kohäsiv haftenden Fixierbinde wird in den noch heißen Kleber von Hand gedrückt und leicht verpresst, so dass die Abschnitte L1 und L2 sich um 5 mm überlappen. Nach Erkalten der Heißklebefuge sind die Abschnitte L1 und L2 fest miteinander zu einem einzigen Streifen verbunden, ohne dass eine Dickstelle erkennbar ist.

Die miteinander verbundenen Abschnitte L1 und L2 werden unter Verwendung einer zylindrischen Papphülse mit einem Durchmesser von 28 mm und einer Wandstärke von 2 mm derart zu einem zylindrischen Bindenkörper aufgewickelt, dass zunächst der Abschnitt L2 auf die Papphülse aufgewickelt wird und nach Erreichen der Verbindungsstelle der Streifen L1 über die Wicklung von L2 weitergewickelt wird, so dass der Abschnitt L1 die äußere Wicklung bildet und sich die dort aufgebrachte Kunststoffbeschichtung wiederum auf der äußeren Lage, d.h. entsprechend auf der Zylinderaußenfläche, befindet. In dieser Form ist die erste innere Binde für die Anwendung fertiggestellt. Die zweite äußere Binde besteht dabei aus zwei Bindenlagen, die nachfolgend mit der Bezeichnung LS und KS bezeichnet sind und beide eine Breiten-Abmessung von 10 cm aufweisen.

Als Bindenlage LS wird eine elastische, kohäsiv haftende Vliesbinde vom Typ 752, Handelsname NOWOPRESS 752, Hersteller: Karl Otto Braun GmbH & Co. KG, Wolfstein, Deutschland als Kompressionsbinde mit Langzugeigenschaften verwendet, wobei das Grundtextil durch Übernähen eines starren Polypropylenvliesstoffs mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben. Diese Bindenlage ist auf beiden Oberflächen mit einem kohäsiv haftenden Klebemittel auf Basis Polyisopren-Kautschuk beschichtet, um den gewünschten kohäsiven Hafteffekt zu erreichen. Die Bindenlage LS (Typ 752) ist dabei wie folgt aufgebaut: 60 % Polypropylen ,12 % Elastan, 28 % IRKautschuk Grund-Vlies: PP-Spunbondvlies , 35 g/qm , thermisch geprägt Nähfaden 133 dtex Elastan (DORLASTAN, BAYER) Nähfadendichte 45 Fäden je 10 cm Breite Nähfaden-Stichlänge, Bindung 3 mm, offene Franse Flächengewicht gedehnt 59 g/qm Elastizität in Längsrichtung (Kettrichtung) Dehnbarkeit / Rückzug nach DIN 61632 160 % / 99 % Haftkraft Seite A/B 60 cN/cm.

Als Typ KS wird ebenfalls die oben spezifizierte in Längsrichtung elastische, kohäsive selbsthaftende Binde auf Basis eines offenporigen Fixierbindengewebes vom Typ 180 verwendet.

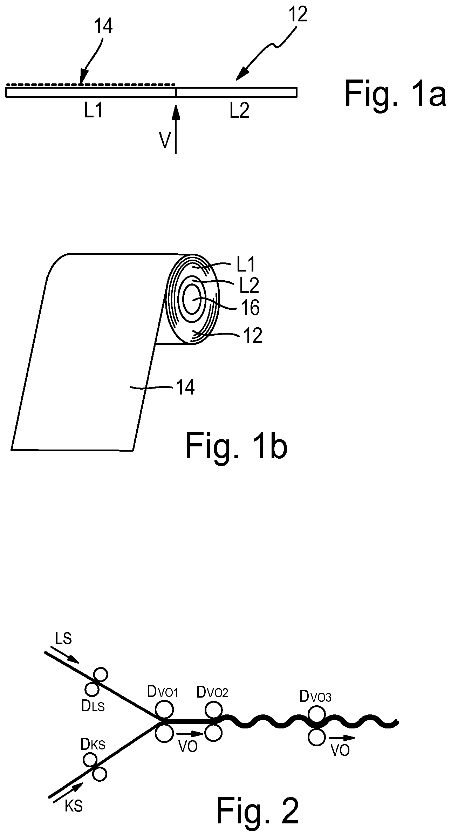

Die Herstellung des erfindungsgemäßen Verbundmaterials zur Bildung der zweiten äußeren Binde erfolgt unter Verwendung getrennter Bahnen der beiden Bindenlagen LS und KS. Das Verfahren ist in Figur 2 dargestellt.

Dazu wird von einer Vorratsrolle das kohäsiv haftende Flächengebilde der Bindenlage LS mit Hilfe eines angetriebenen ersten Quetschwalzenpaares DLS mit einer Transportgeschwindigkeit von 5 m/min abgezogen, wobei möglichst geringe Zugkräfte verwendet werden, um den ungedehnten Zustand der Bindenlage weitgehend zu erhalten. Die Breite der Quetschwalzen beträgt 15 cm. Mit Hilfe eines angetriebenen zweiten Quetschwalzenpaares DKS wird die Bahn der zweiten Bindenlage KS getrennt von der Bahn der ersten Bindenlage mit der Transportgeschwindigkeit von 5 m/min eingezogen, wobei die Bahnen im Wesentlichen kantengerade übereinander in der gleichen Transportrichtung geführt werden. Die beiden Quetschwalzenpaare haben die Funktion eines Klemmpunktes, d. h. der Transport der Materialbahnen erfolgt schlupffrei.

Beide Bahnen werden anschließend zwischen einem angetriebenen dritten Quetschwalzenpaar DVO1 und gegebenenfalls einem weiteren Quetschwalzenpaar DV02 kantengerade zusammengeführt und mit einem Anpressdruck von 3 bar zwischen den Walzen zu einem Lagenverbund permanent haftend miteinander zu einem Laminat verbunden, wobei die Geschwindigkeit 9 m/min, d. h. dass 1,9-Fache der Einzugsgeschwindigkeit beträgt. Dadurch wird ein Verzug der Bahnen der ersten und zweiten Bindenlage in Längsrichtung auf einen Dehnungszustand D_{fix} gleich 80% erreicht, welcher die erfindungsgemäße Dehnungsbegrenzung des Verbundes aus den beiden Bindenlagen LS und KS herstellt. Anschließend wird der Verbund, der die zweite äußere Binde bildet, durch das angetriebene Quetschwalzenpaar DVO3 geführt, welches so eingerichtet wird, dass die Transportgeschwindigkeit wieder die Ursprungsgeschwindigkeit von 5 m/min aufweist.

Dadurch wird der Verzug der miteinander verbundenen Bindenlagen aufgehoben und der ursprünglich ungedehnte Zustand annähernd wiederhergestellt. Auf diese Weise entsteht ein flaches Laminat mit zwei verschiedenen Oberflächen, also zwei unterschiedlichen Seiten A und B (s. Angaben zur Haftkraft). Seite A wird aus der ersten Bindenlage LS gebildet, Seite B aus der zweiten Bindenlage KS, wobei es von Hand beinahe nicht mehr gelingt, die ursprünglichen Bindenlagen voneinander zu trennen, weil sie fest haftend miteinander verpresst sind.

Die Eigenschaften des Verbundes der zweiten äußeren Binde ergeben sich aus der nachstehenden Tabelle: Materialaufbau 35 % Gewebe Typ 180 ,65 % Nonwoven Typ 752 Flächengewicht gedehnt (DIN 61632) 128 g/qm Elastizität in Längsrichtung (Kettrichtung) Dehnbarkeit / Rückzug nach DIN 61632 78 % / 98 % Luftdurchlässigkeit DIN 53887 200 l/qm sec Haftkraft Seite A/B 38 cN/cm

Das Laminat wird anschließend in der gewünschten Länge, z. B. 750 cm ungedehnt, auf eine Hartpapierhülse zur fertigen Kompressionsbinde mit Dehnungsbegrenzung aufgewickelt und dient dann in der Zusammenstellung als zweite äußere Binde.

Weitere Vorteile und Merkmale der Erfindung ergeben sich aus den übrigen Anmeldungsunterlagen, insbesondere der nachstehend beschriebenen Ausführungsform. Dabei zeigen:
- Figur 1: in den Darstellungen a) und b) eine erfindungsgemäße erste innere Binde der Kompressionsbindenzusammenstellung
- Figur 2: ein Herstellungsverfahren für eine erfindungsgemäße zweite äußere Binde der Kompressionsbindenzusammenstellung, und
- Figur 3: ein Kraft-Dehnungsdiagramm der zweiten äußeren Binde.

Figur 1 zeigt in Darstellung a) eine erste innere Binde, die in ihrer Gesamtheit mit dem Bezugszeichen 12 versehen ist und aus einem ersten Bindenabschnitt L1 sowie einem zweiten Bindenabschnitt L2 gebildet wird, die an einer Verbindungsstelle V auf Stoß aneinander angesetzt sind und miteinander so verbunden sind, dass sie durch beim Anwickeln entstehenden Kräfte nicht voneinander getrennt werden. Auf einer Seite des Bindenabschnitts L1 ist eine Antirutschbeschichtung 14 aus Kunststofffragmenten aufgebracht, die eine diskontinuierliche offenporige Schicht bilden, so dass die Atmungsaktivität des Abschnitts L1 vergleichsweise wenig eingeschränkt wird. Dabei wird die Binde 12 mit dem Abschnitt L1 und hier mit der Kunststoffbeschichtung 14 zuerst auf die Haut einer Extremität aufgewickelt.

Darstellung b) zeigt nun eine auf einen Hülsenkern 16 aufgewickelte erste Binde 12, wobei die äußere Wicklung durch den Abschnitt L1 und die innere Wicklung durch den Abschnitt L2 gebildet ist. Dabei erfolgt die Aufwicklung so, dass die mit der Kunststoffschicht 14 versehene Seite der Binde L1 nach außen weist und so besonders einfach auf eine Extremität aufgewickelt werden kann.

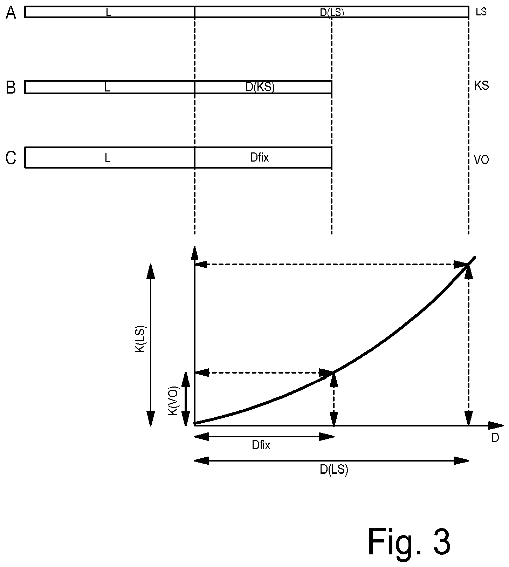

Figur 3 zeigt ein Kraftdehnungsdiagramm der äußeren zweiten Binde bestehend aus einer Bindenlage LS, einer Bindenlage KS, wobei mit VO die gesamte Binde aus den beiden Bindenlagen bezeichnet ist. Hierbei ist zu erkennen, dass die maximale Dehnung D_{fix} der Dehnung der Binde KS entspricht, wobei die Dehnungskraft dem Verlauf der Binde LS entspricht.

## Patentansprüche

1. Kompressionsbindenzusammenstellung umfassend eine erste innere Binde (12) sowie eine zweite äußere Binde, wobei die zweite äußere Binde im Gebrauch über die erste innere Binde (12) anlegbar ist, wobei die erste innere Binde einen ersten Abschnitt (L1) umfasst, der zumindest auf seiner der Haut eines Trägers zugewandten Seite eine Polsterschicht aufweist und einen an den ersten Abschnitt (L1) anschließenden, elastischen und kohäsiv haftenden zweiten Abschnitt (L2) und wobei die zweite äußere Binde elastisch und kohäsiv haftend ausgebildet ist, **dadurch gekennzeichnet, dass** die zweite äußere Binde mindestens zwei miteinander verbundene Bindenlagen (LS, KS) umfasst, die im separierten Zustand voneinander verschiedene elastische Eigenschaften aufweisen.

2. Kompressionsbindenzusammenstellung nach Anspruch 1 , **dadurch gekennzeichnet, dass** auf der der Haut zugewandten Seite der Polsterschicht der ersten inneren Binde (12) eine diskontinuierliche, insbesondere offenporige Kunststoffbeschichtung (14) aufgebracht ist.

3. Kompressionsbindenzusammenstellung nach einem der vorangehenden Ansprüche, **dadurch gekennzeichnet, dass** die beiden Abschnitte (L1, L2) der ersten inneren Binde (12) miteinander verbunden sind und in Längsrichtung der Binde (12) aneinander anschließen.

4. Kompressionsbindenzusammenstellung nach einem der vorangehenden Ansprüche, **dadurch gekennzeichnet, dass** die Polsterschicht als Polsterwatte, insbesondere aus Baumwolle, Viskose, Polyester oder Polypropylen oder Kombinationen hiervon, gebildet ist und insbesondere eine Vliesschicht ist.

5. Kompressionsbindenzusammenstellung nach einem der vorangehenden Ansprüche, **dadurch gekennzeichnet, dass** die Polsterschicht den ersten Bindenabschnitt (L1) bildet.

6. Kompressionsbindenzusammenstellung nach einem der vorangehenden Ansprüche, **dadurch gekennzeichnet, dass** die mindestens zwei Bindenlagen (LS, KS) kohäsiv haftend und elastisch ausgebildet sind, wobei eine erste Bindenlage (LS) eine Dehnbarkeit gemessen nach DIN 61632 von D1 = 100%-180% aufweist und die zweite Bindenlage (KS) einen Dehnbarkeit gemessen nach DIN 61632 von D2 = 10% -100%.

7. Kompressionsbindenzusammenstellung nach Anspruch 6, **dadurch gekennzeichnet, dass** die mindestens zwei Bindenlagen (LS, KS) so miteinander verbunden sind, dass sich eine Dehnbarkeit der zweiten äußeren Binde gemessen nach DIN 61632 von D_{fix} = 40%- 80% ergibt.

8. Kompressionsbindenzusammenstellung nach Anspruch 7, **dadurch gekennzeichnet, dass** die erste (12) und die zweite Binde sich vollflächig und insbesondere kantengenau überdecken und über den gesamten Dehnungsbereich 0 bis Dfix haftend miteinander verbunden sind.

## Claims

1. Compression bandage assembly, comprising a first inner bandage (12) as well as a second outer bandage, the second outer bandage being attachable in use over the first inner bandage (12), wherein the first inner bandage comprises a first section (L1) having a padding layer at least on its side facing the skin of a bearer, and an elastic and cohesively adhering second section (L2) continuous to the first section (L1), and wherein the second outer bandage is made elastic and cohesively adhering, **characterized in that** the second outer bandage comprises at least two bandage layers (LS, KS) connected to each other which, when separated, have elastic properties different from each other.

2. Compression bandage assembly as claimed in claim 1, **characterized in that** a discontinuous, particularly open-porous plastics coating (14) is applied on the skin-facing side of the padding layer of the first inner bandage (12).

3. Compression bandage assembly as claimed in one of the preceding claims, **characterized in that** the two sections (L1, L2) of the first inner bandage (12) are connected to each other and are contiguous in the longitudinal direction of the bandage (12).

4. Compression bandage assembly as claimed in one of the preceding claims, **characterized in that** the padding layer is formed as a wadding, particularly made of cotton, viscose, polyester or polypropylene or a combination thereof, and is particularly a nonwoven layer.

5. Compression bandage assembly as claimed in one of the preceding claims, **characterized in that** the padding layer forms the first bandage section (L1).

6. Compression bandage assembly as claimed in one of the preceding claims, **characterized in that** the at least two bandage layers (LS, KS) are formed in a cohesively adhering and elastic manner, wherein the first bandage layer (LS) has an extensibility of D1 = 100% - 180% as measured according to DIN 61632 and the second bandage layer (KS) has an extensibility of D2 = 10% - 100% as measured according to DIN 61632.

7. Compression bandage assembly as claimed in claim 6, **characterized in that** the at least two bandage layers (LS, KS) are connected to each other in such a way that an extensibility of the second outer bandage of D_{fix} = 40% - 80% as measured according to DIN 61632 is achieved.

8. Compression bandage assembly as claimed in claim 7, **characterized in that** the first (12) and the second bandage overlap each other over the entire surface and particularly with aligned edges and are adhesively connected to each other over the entire range of extensibility 0 to Dfix.

## Revendications

1. Ensemble de bandages de compression comprenant un premier bandage intérieur (12) ainsi qu'un deuxième bandage extérieur, dans lequel le deuxième bandage extérieur peut être placé sur le premier bandage intérieur (12) lors de l'usage, dans lequel le premier bandage intérieur comprend une première section (L1) qui présente une couche de rembourrage au moins sur sa face montrant vers la peau d'un porteur ainsi qu'une deuxième section (L2) qui est contiguë à la première section (L1), est élastique et adhère de façon cohésive, et dans lequel le deuxième bandage extérieur est conçu de manière élastique et à adhérer de façon cohésive, **caractérisé par le fait que** le deuxième bandage extérieur comprend au moins deux couches de bandage (LS, KS) reliées entre elles qui, à l'état séparé, présentent des propriétés élastiques différentes les unes des autres.

2. Ensemble de bandages de compression selon la revendication 1, **caractérisé par le fait qu'**un revêtement de matière plastique (14) discontinu, en particulier à pores ouverts, est appliqué sur la face de la couche de rembourrage du premier bandage intérieur (12), qui montre vers la peau.

3. Ensemble de bandages de compression selon l'une quelconque des revendications précédentes, **caractérisé par le fait que** les deux sections (L1, L2) du premier bandage intérieur (12) sont reliées l'une à l'autre et se rejoignent dans le sens longitudinal du bandage (12) .

4. Ensemble de bandages de compression selon l'une quelconque des revendications précédentes, **caractérisé par le fait que** la couche de rembourrage est réalisée en tant que ouate de rembourrage, en particulier en coton, viscose, polyester ou polypropylène ou en combinaisons de ceux-ci, et, en particulier, est une couche de non-tissé.

5. Ensemble de bandages de compression selon l'une quelconque des revendications précédentes, **caractérisé par le fait que** la couche de rembourrage forme la première section de bandage (L1).

6. Ensemble de bandages de compression selon l'une quelconque des revendications précédentes, **caractérisé par le fait que** lesdites au moins deux couches de bandage (LS, KS) sont conçues de manière à adhérer de façon cohésive et élastique, dans lequel une première couche de bandage (LS) présente une extensibilité, mesurée selon DIN 61632, de D1 = 100 % -180 %, et la deuxième couche de bandage (KS) présente une extensibilité, mesurée selon DIN 61632, de D2 = 10 % - 100 %.

7. Ensemble de bandages de compression selon la revendication 6, **caractérisé par le fait que** lesdites au moins deux couches de bandage (LS, KS) sont reliées entre elles de telle sorte que l'on obtienne une extensibilité du deuxième bandage extérieur, mesurée selon de DIN 61632, de D_{fix} = 40 % - 80 %.

8. Ensemble de bandages de compression selon la revendication 7, **caractérisé par le fait que** les premier (12) et deuxième bandages se recouvrent sur toute la surface et en particulier à bords précis et sont reliés de manière adhésive l'un à l'autre sur l'ensemble de la plage d'extension 0 à Dfix.
